# EUROPEAN PATENT SPECIFICATION

(11) **EP 1 804 597 B1**
(45) Date of publication and mention of the grant of the patent: **01.08.2012**
(21) Application number: 05764556.6
(22) Date of filing: 30.06.2005
(51) Int. Cl.: A41B 9/02, A61F 13/15

(54) **PROCESS OF MAKING BOXER SHORTS FROM A WEB**
VERFAHREN ZUR HERSTELLUNG VON BOXER-SHORTS AUS EINER BAHN
PROCEDE POUR FABRIQUER UN CALEÇON-BOXER A PARTIR D'UN LE DE TISSU

(30) Priority: 30.09.2004 US 954656; 30.09.2004 US 954628
(43) Date of publication of application: 11.07.2007
(73) Proprietor: KIMBERLY-CLARK WORLDWIDE, INC., Neenah, WI 54956 (US)
(72) Inventor: FRANKE, Mark, Steven, Neenah, Wisconsin 54956 (US); MORTELL, Heather, Schenck, Neenah, Wisconsin 54956 (US); COENEN, Joseph, Daniel, Kaukauna, Wisconsin 54130 (US); POPP, Robert, Lee, Hortonville, Wisconsin 54944 (US); RATLIFF, Kathleen, Irene, Neenah, Wisconsin 54956 (US)
(74) Representative: Davies, Christopher Robert
(86) International application number: PCT/US2005/023662
(87) International publication number: WO 2006/038946

(56) References cited:
- WO-A-03/041625
- WO-A-2004/052131
- US-A- 4 327 448
- US-A- 5 554 149
- US-A1- 2004 098 791
- US-A1- 2004 116 881

## Description

### BACKGROUND OF THE INVENTION

This invention is directed to methods of making pants having side seams and a contracted crotch region. More particularly, the invention pertains to methods of making boxer shorts having side seams and a contracted crotch region. The boxer shorts may be absorbent or non-absorbent.

Pant-like garments have numerous applications including disposable clothing, training pants, feminine care products, adult incontinence products, disposable swimwear, or the like. Pant-like disposable garments are typically three-dimensional products with closed sides so that the product has a unitary waist opening and two leg openings. The wearer raises and lowers the garment to apply the product. Three-dimensional, boxer shorts-like products are particularly appealing because the boxer shorts look more like conventional articles of clothes.

Many disposable pants are formed as composite structures in which several components are combined to form a product specifically suited to its intended purpose. For example, disposable pants often include one or more absorbent materials intended to absorb various bodily exudates such as urine, menstrual fluid, and/or sweat. Such products may include a liquid permeable bodyside liner and a liquid impermeable outer cover, and can include other materials and features such as elastic materials and containment structures.

However, many disposable pants are aesthetically unappealing. Existing disposable absorbent pants can be overly bulky and often resemble disposable baby diapers. Various attempts have been made to provide disposable pants having an improved, more clothing-like appearance. However, disposable pants, particularly disposable absorbent boxer shorts, present many manufacturing challenges. In part, this is due to the high speed that is necessary to economically produce relatively low-cost disposable absorbent products. Product design is often compromised by cost and manufacturing constraints, resulting in disposable pants that lack aesthetic appeal and product function. In addition, crotch depth is required for a good fit, but difficult to achieve in a garment like boxer-shorts with hanging legs when using conventional manufacturing processes.

There is thus a need or desire for garment-like, aesthetically appealing boxer shorts, as well as methods of efficiently manufacturing such boxer shorts.

A prior art part, having the features of the preamble of claim 1, is shown in WO 2004/052131.

### SUMMARY OF THE INVENTION

According to the present invention, there is provided a part as claimed in claim 1 and a method as claimed in claim 5. In response to the discussed difficulties and problems encountered in the prior art, new pants, and methods for manufacturing such pants, have been invented. The material for the garment shell of the pant is handled as a single web, or a continuous web of multiple pants, throughout assembly until seaming in order to streamline the assembly. The pants can include an absorbent assembly and can be made in either the machine direction or the cross direction.

One aspect of the invention pertains to a method of making a pant having side seams and hanging legs, the method comprising providing a web; contracting the web in one or more selected areas; cutting at least one portion of the web to define leg openings; and attaching a first region and a second region together to form the side seams.

The web may be folded against a support structure. Examples of suitable support structures include internal support structures such as bars over which the web may be folded, or external support structures such as opposing vacuum conveyors between which the web may be folded. Additionally, the web may be contracted in the crotch region, or a strip applied to the crotch region, while the web is folded against the support structure. For instance, a strip may be applied to the web against the folded portion of the web while the web is on the support structure. The web may also be cut while on the support structure. In certain embodiments, a multi-lane production system may be used, in which case the web is folded against at least two support structures each parallel to a direction in which the web is conveyed, and each machine-direction array of pant assemblies is folded against a single support structure.

An absorbent structure may also be attached to the web. The absorbent structure may be attached to the web while the web is folded, prior to folding the web, or after unfolding the web.

The leg openings are formed from a slit along each of the transversely opposed edges of the web. The slit may be a single slit or a T-shaped slit. Any of the slits may include a circular cut-out at the interior end of the leg opening to reduce the stress concentration.

The invention relates to a wide variety of absorbent and non-absorbent pants, including training pants, swim pants, diaper pants, incontinence garments, feminine care products, health care garments, apparel for institutional, industrial, and consumer use, or other garments. Disposable absorbent pants are adapted to be worn adjacent to the body of a wearer to absorb and contain various exudates discharged from the body.

### BRIEF DESCRIPTION OF THE DRAWINGS

These and other objects and features of this invention will be better understood from the following detailed description taken in conjunction with the drawings, wherein:
Fig. 1 is a front view of one embodiment of a pant according to the invention.
Figs. 1A and 1B illustrate dimensions described with respect to Tables 1 and 2.
Fig. 2A is a perspective cut-away view of one embodiment of a pant according to the invention.
Fig. 2B is a perspective cut-away view of one embodiment of a pant according to the invention.
Fig. 3A is a plan view of the garment shown in Fig. 2A, showing the side facing the wearer.
Fig. 3B is a plan view of the garment shown in Fig. 2B, showing the side facing the wearer.
Fig. 3C is a plan view of the garment shown in Fig. 2A, showing the side facing the wearer without an absorbent structure.
Figs. 3D-3G are plan views of garments similar to the garment shown in Fig. 3C but with alternative strip configurations in the contracted crotch region.
Fig. 4 is a top view of a web.
Fig. 5 is a top view of the web of Fig. 4 including leg openings and strips applied to the web for assembling pants using a machine direction assembly.
Figs. 5A and 5B illustrate dimensions described with respect to Tables 1 and 2.
Fig. 6 is a top view of the web of Fig. 5 after contraction of the web.
Fig. 7 is a side view of a looper drum for applying an elastic strip to the web.
Fig. 8A is a side view of a process for applying a strip to the web.
Fig. 8B is a perspective view of a machine direction process for assembling pants.
Fig. 8C is a top view of a multi-lane machine direction process for assembling pants.
Fig. 8D is a cross-sectional view taken along line 8-8 in Fig. 8C.
Fig. 9 is a side view of a corrugating drum for corrugating the web of Fig. 5.
Fig. 10A is a top view of the web of Fig. 4 including leg openings and strips applied to the web for assembling pants using a cross direction assembly.
Fig. 10B is a perspective view of a cross direction process for assembling pants.
Fig. 10C is a cross-sectional view taken along line 10-10 in Fig. 10B.
Fig. 10D is a top view of a multi-lane cross direction process for assembling pants.
Fig.11 is a top view of the web of Fig. 10A after contraction of the web.
Fig. 12 is a side view of the web of Fig. 10A passing through corrugating rollers for corrugating the web of Fig. 10A.
Figs. 13A-13L are top views of the web having various leg openings.

### DEFINITIONS

Within the context of this specification, each term or phrase below will include the following meaning or meanings.

"Attached" refers to the joining, adhering, connecting, bonding, or the like, of two elements. Two elements will be considered to be attached together when they are attached directly to one another or indirectly to one another, such as when each is directly attached to intermediate elements.

"Bonded" refers to the joining, adhering, connecting, attaching, or the like, of two elements. Two elements will be considered to be bonded together when they are bonded directly to one another or indirectly to one another, such as when each is directly bonded to intermediate elements.

"Boxer shorts" refers to a garment having hanging legs.

"Coform" is a composite material that is essentially an air-formed matrix of thermoplastic polymer microfibers, including meltblown fibers, and a multiplicity of individualized cellulose and/or staple fibers and/or particulates such as superabsorbents disposed throughout the matrix of microfibers and engaging at least some of the microfibers to space the microfibers to intertwine and hold captive within the matrix of microfibers by mechanical entanglement of the microfibers with the cellulose and/or staple fibers and/or particulates including superabsorbent

"Comprising" is inclusive or open-ended and does not exclude additional, unrecited elements or method steps.

"Connected" refers to the joining, adhering, bonding, attaching, or the like, of two elements. Two elements will be considered to be connected together when they are connected directly to one another or indirectly to one another, such as when each is directly connected to intermediate elements.

"Corrugated" refers to the condition of a material which has been gathered into pleats or regular rugosities or folds, the material being shortened thereby.

"Cut-out" refers to a cut portion that includes one portion of a web removed from a remainder of the web, as opposed to a "slit," which is a cut in a web that does not result in the removal of any portion of the web.

"Disposable" refers to articles which are designed to be discarded after a limited use rather than being laundered or otherwise restored for reuse.

"Elastic," "elasticized," and "elasticity" mean that property of a material or composite by virtue of which it tends to recover its original size and shape after removal of a force causing a deformation.

"Elastomeric" refers to a material or composite which can be elongated by at least 25 percent of its relaxed length and which will recover, upon release of the applied force, at least 10 percent of its elongation. It is generally preferred that the elastomeric material or composite be capable of being elongated by at least 100 percent, more preferably by at least 300 percent, of its relaxed length and recover, upon release of an applied force, at least 50 percent of its elongation.

"Fabric" is used to refer to all woven, knitted and nonwoven fibrous webs.

"Flat web" comprises any material used for making garments that can be provided and processed in a substantially open, unfolded state; while the web can possess ripples or areas that do not lie exactly within an overall plane of the web, all points of the web should be reasonably identifiable as constituents in either an upper or a lower surface of the web. No portions of a flat web are enclosed or fixed into a loop or tunnel-like, or three-dimensional configuration.

"Garment shell" refers to an outer cover or outer layer of a garment. In a single-ply garment, the single layer of the garment is the garment shell.

"Garment insert" refers to an inner layer of a garment. The garment insert provides a close-to-the-body fit about a wearer's lower torso, thereby serving as a form of built-in underwear within the garment.

"Hanging legs" refers to the portions of a garment which extend from the crotch region downward to the leg openings. "Downward" refers to a direction toward the ground when the garment is positioned on a standing wearer.

"Hydrophilic" describes fibers or the surfaces of fibers which are wetted by aqueous liquids in contact with the fibers. The degree of wetting of the materials can, in turn, be described in terms of the contact angles and the surface tensions of the liquids and materials involved. Equipment and techniques suitable for measuring the wettability of particular fiber materials or blends of fiber materials can be provided by a Cahn SFA-222 Surface Force Analyzer System, or a substantially equivalent system. When measured with this system, fibers having contact angles less than 90 degrees are designated "wettable" or hydrophilic, while fibers having contact angles greater than 90 degrees are designated "nonwettable" or hydrophobic.

"Integral" is used to refer to various portions of a single unitary element rather than separate structures bonded to or placed with or placed near one another.

"Layer" when used in the singular can have the dual meaning of a single element or a plurality of elements.

"Liquid impermeable," when used in describing a layer or multi-layer laminate, means that a liquid, such as urine, will not pass through the layer or laminate, under ordinary use conditions, in a direction generally perpendicular to the plane of the layer or laminate at the point of liquid contact. Liquid, or urine, may spread or be transported parallel to the plane of the liquid impermeable layer or laminate, but this is not considered to be within the meaning of "liquid impermeable" when used herein.

"Machine direction" refers to the length of a fabric in the direction in which it is produced, as opposed to "cross direction" which refers to the width of a fabric in a direction generally perpendicular to the machine direction.

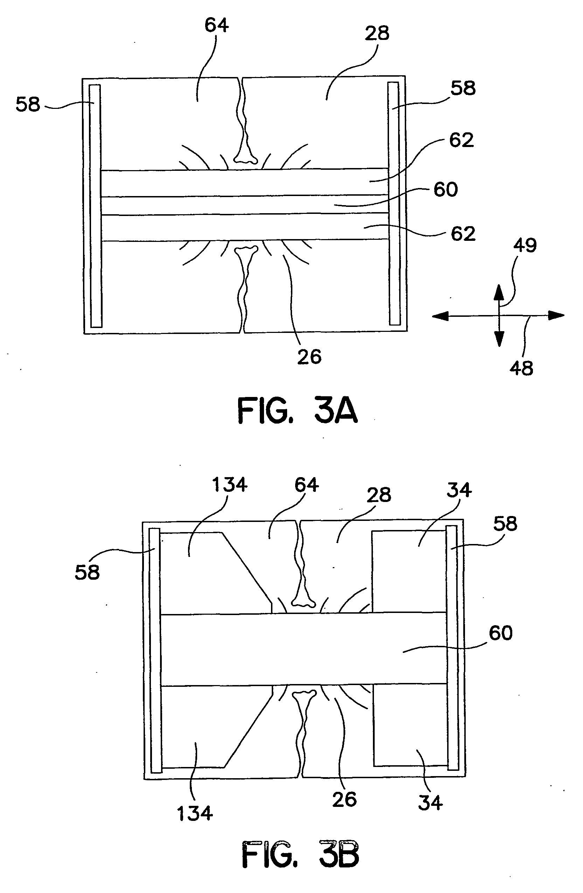

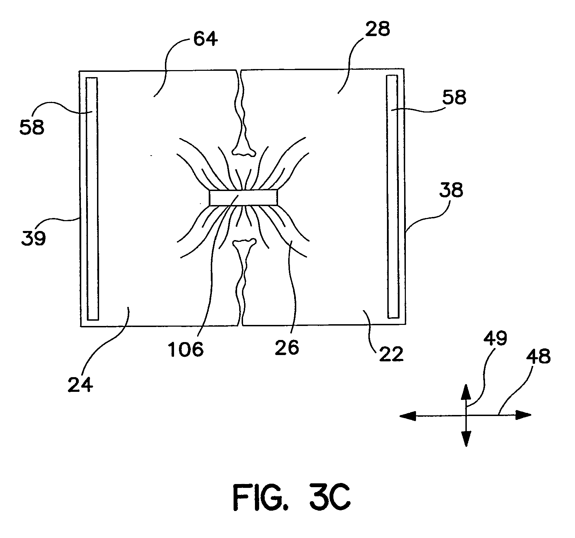

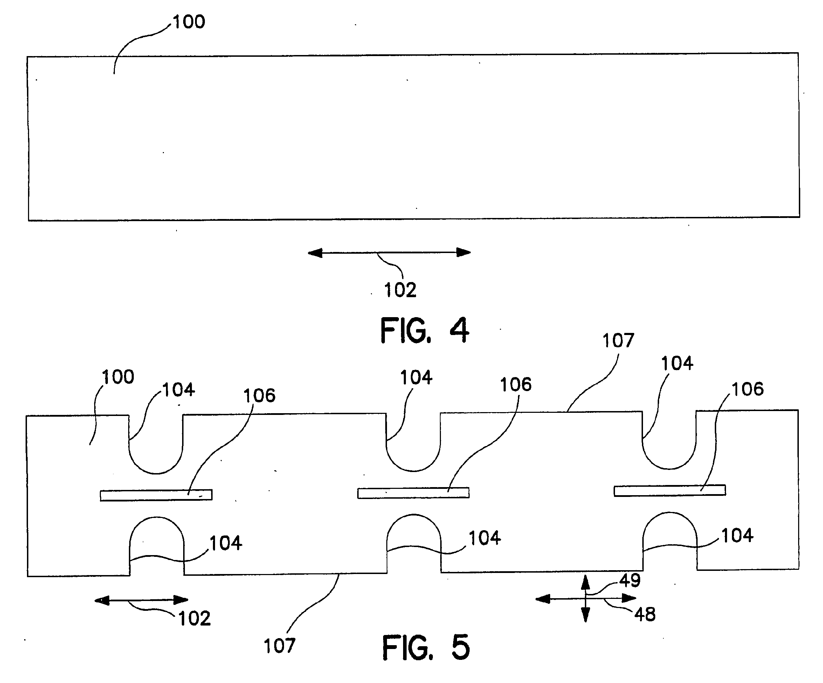

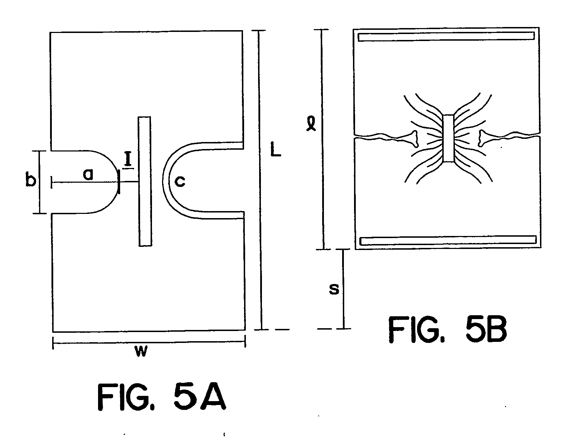

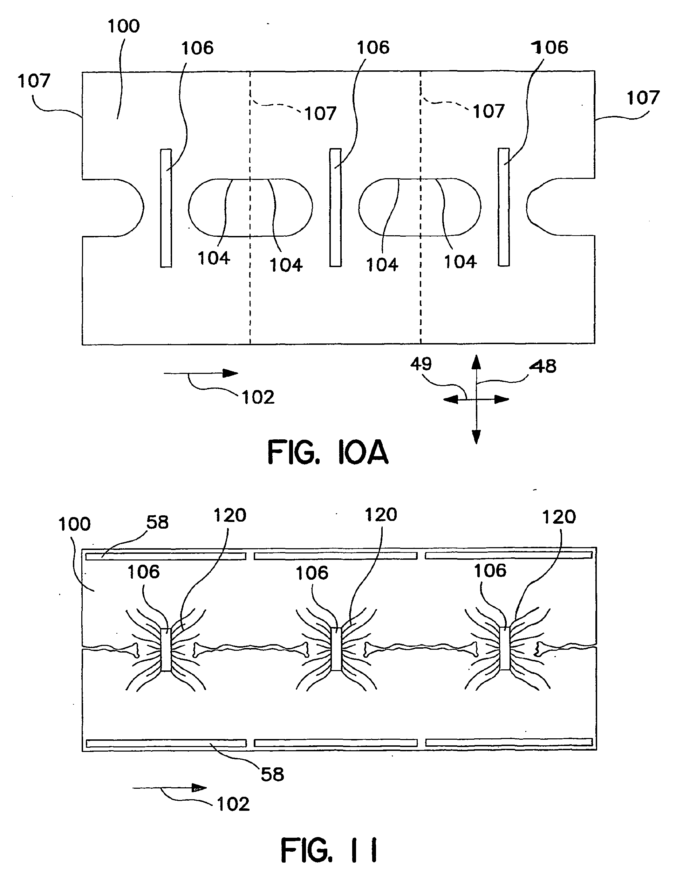

The term "machine direction assembly" refers to a manufacturing process in which disposable products travel in an end-to-end or waist-to-waist orientation. A process utilizing a machine direction assembly entails products traveling in a machine direction through a converting machine with their longitudinal axes 48 (Figs. 3A, 3C) parallel to the direction of arrow 102 (Fig. 5). "Cross direction assembly" entails the products traveling in a machine direction in a side-by-side orientation with their lateral axes 49 (Figs. 3A, 3C) parallel to the direction of arrow 102, such as is illustrated in Fig. 10A.

"Member" when used in the singular can have the dual meaning of a single element or a plurality of elements.

"Nonwoven" and "nonwoven web" and "web" refer to materials and webs of material which are formed without the aid of a textile weaving or knitting process.

"Operatively joined," with reference to the attachment of an elastic member to another element, means that the elastic member can be attached to or connected to the element, and can additionally be treated with heat or chemicals, by pre-stretching, or the like, to give the element elastic properties; and with reference to the attachment of a non-elastic member to another element, means that the member and element can be attached in any suitable manner that permits or allows them to perform the intended or described function of the joinder. The joining, attaching, connecting or the like can be either directly, such as joining either member directly to an element, or can be indirectly by means of another member disposed between the first member and the first element.

The term "spunbonded fibers" refers to small diameter fibers which are formed by extruding molten thermoplastic material as filaments from a plurality of fine capillaries of a spinnerette having a circular or other configuration, with the diameter of the extruded filaments then being rapidly reduced as by, for example, in U.S. Patent 4,340,563 to Appel et al., U.S. Patent 3,692,618 to Dorschner et al., U.S. Patent 3,802,817 to Matsuki et al., U.S. Patents 3,338,992 and 3,341,394 to Kinney, U.S. Patent 3,502,763 to Hartman, U.S. Patent 3,502,538 to Petersen, and U.S. Patent 3,542,615 to Dobo et al. Spunbond fibers are quenched and generally not tacky on the surface when they enter the draw unit, or when they are deposited onto a collecting surface. Spunbond fibers are generally continuous and may have average diameters larger than 7 microns, often between about 10 and 30 microns.

"Stretchable" means that a material can be stretched, without breaking, by at least 50% (to 150% of its initial (unstretched) length) in at least one direction, suitably by at least 100% (to 200% of its initial length), desirably by at least 150% (to at least 250% of its initial length).

"Surface" includes any layer, film, woven, nonwoven, laminate, composite, or the like, whether pervious or impervious to air, gas, and/or liquids.

"Three-dimensional garment" refers to a garment that cannot be laid flat with all of its seams in one plane.

These terms may be defined with additional language in the remaining portions of the specification.

### DESCRIPTION OF PREFERRED EMBODIMENTS

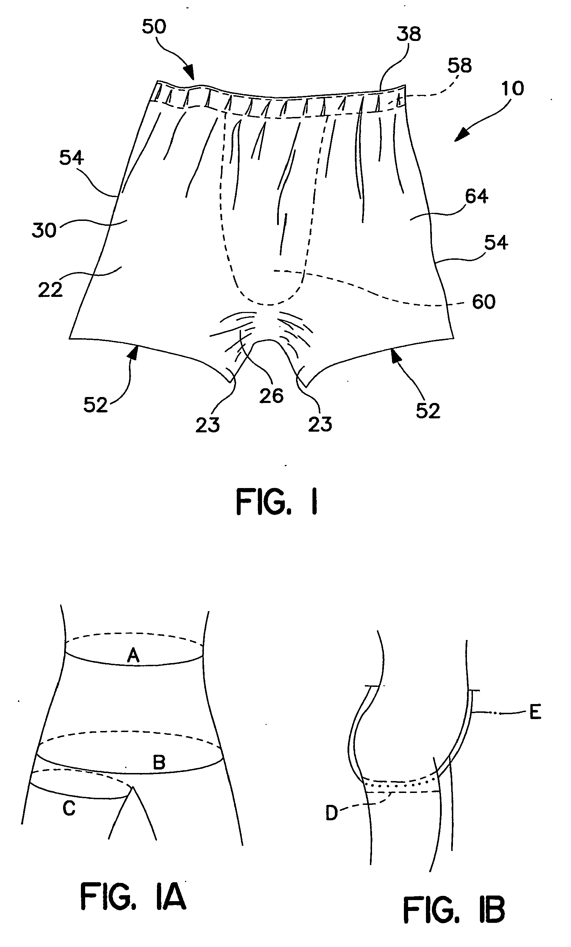

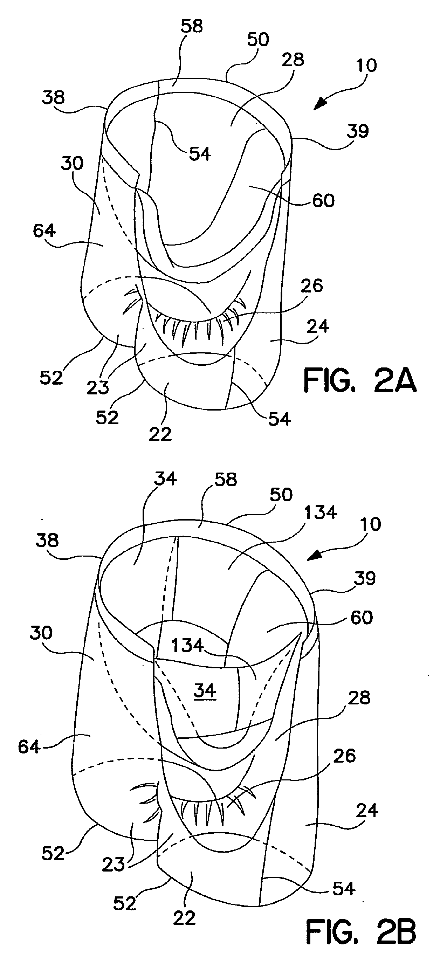

As representatively illustrated in Figs. 1, 2A, and 2B, an embodiment of a pant 10 of the invention includes a garment shell 64. The garment shell 64 can include a front region 22, a back region 24, a contracted crotch region 26, an inner surface 28 which is configured to contact the wearer, and an outer surface 30 opposite the inner surface 28 which is configured to contact the wearer's clothing. The pant 10 also defines a pair of longitudinally opposed waist edges, which are designated front waist edge 38 and back waist edge 39. The front region 22 is contiguous with the front waist edge 38, and the back region 24 is contiguous with the back waist edge 39. The front region 22 includes the portion of the pant 10 which, when worn, is positioned on the front of the wearer while the back region 24 includes the portion of the pant 10 which, when worn, is positioned on the back of the wearer. The contracted crotch region 26 of the pant 10 includes the portion of the pant which, when worn, is positioned between the legs of the wearer and covers the lower torso of the wearer. As illustrated in Figs. 1, 2A, and 2B the front and back regions 22 and 24 are joined together at side seams 54 to define a three-dimensional pant configuration having a waist opening 50 and a pair of leg openings 52. The contracted crotch region 26 may be positioned approximately transversely midway between the leg openings 52 and aligned with a longitudinal centerline of the garment shell 64. In particular embodiments, the pant 10 can include an absorbent structure 60.

The garment shell 64 includes a contracted crotch region 26. As described more fully below, the contraction of the contracted crotch region 26 can be accomplished either elastically or inelastically. The contraction of crotch region 26 provides crotch depth that provides a good fit through the contracted crotch region 26, thereby allowing the front and back regions to hang properly. The garment shell 64 can also include hanging legs 23 which extend front the contracted crotch region 26 downward to the leg openings 52 (Figs. 1, 2A, and 2B).

The pant 10 also includes side seams 54 that connect the front region 22 to the back region 24 to create the pant 10. The side seams 54 can take any number of forms, including both refastenable and non-refastenable seams, as are known in the art. The provision of the side seams 54 can be accomplished in the manner described in U.S. Patent 6,192,521 issued 27 February 2001 to Alberts et al.; U.S. Patent 5,046,272, issued 10 September 1991 to Vogt et al., or in the manner described in U.S. Patent 6,565,691, issued 20 May 2003 to Tornsovic, et al.; U.S. Patent 6,723,034 issued 20 April 2004 to Dutrance, et al.; U.S. Patent 6,596,113 issued 22 July 2003 to Csida, et al.; and/or U.S. Patent 6,513,221 issued 04 February 2003 to Vogt, et al. As is known in the art, the side seams 54 can be inward or outward fin seams or lap seams (not shown).

The pant 10 can also have a waist elastic member 58 extending along at least a portion of the front waist edge 38 and/or the back waist edge 39. The waist elastic member 58 can be formed of any suitable elastic material. As is well known to those skilled in the art, suitable elastic materials include sheets, strands, or ribbons of natural rubber, synthetic rubber, or thermoplastic elastomeric polymers. The elastic materials can be stretched and adhered to a substrate, adhered to a gathered substrate, or adhered to a substrate and then elasticized or shrunk, for example with the application of heat, such that elastic constrictive forces are imparted to the substrate. In one particular embodiment, for example, the waist elastic member 58 includes a plurality of dry-spun coalesced multifilament spandex elastomeric threads sold under the trade name LYCRA® and available from Invista Corporation, Wilmington, Delaware, U.S.A. Alternatively, multiple strands of 310 decitex LYCRA® may be also laminated at 250% elongation between spunbond facings in addition to an adhesive.

As another alternative, the waist elastic member 58 can be a material exhibiting delayed retraction, or can in fact be non-elastic. Delayed retraction materials may include those designed to retract relatively slowly following compression, such as "temporarily inhibited" elastic materials. "'Temporarily inhibited" materials are described, for example, in U.S. Patent No. 5,545,158 issued August 13, 1996, to Jessup, U.S. Patent No. 5,669,996 issued September 23, 1997, to Jessup, and U.S. Patent No. 5,500,063 issued March 19, 1996, to Jessup, and references cited therein. Alternatively, a delayed retraction material may be designed to resist retraction until an activation process occurs, such as so-called "latent elastic" materials. Suitable retractive materials for use as a delayed retraction material can alternatively comprise any material adapted to retract upon activation, whether immediately upon activation or subsequently thereto. The retractive material may include elastomeric or nonelastomeric materials. Suitable nonelastomeric retractive materials may include without limitation polyether block amides (PEBAX®) or the like, and laminates thereof. Suitable elastomeric retractive materials may include without limitation LYCRA® materials, elastomeric materials including latex or rubber or synthetic urethanes, or the like, and laminates thereof. In particular embodiments, the retractive material may include an elastomeric material having an unstable state relative to some other stable and elastic state. In such embodiments, the retractive material can, but need not, have elastomeric properties in the unstable state. Other examples include heat-shrinkable elastic materials such as described in U.S. Patent No. 4,816,094 issued March 28, 1989 to Pomplun et aL, U.S. Patent No. 4,665,306 issued May 12, 1987 to Roland et aL, and U.S. Patent No. 4,663,106 issued May 5, 1987 to Pomplun et al.

A pant of this type can be designed to fit wearers in a wide range of sizes by adjusting the pant dimensions based on the anthropometric features of an intended wearer. Ratios of wearer dimensions to pant dimensions for a suitable boxer-style pant have been determined and are shown in Table 1. In addition, stylistic variations such as hip-hugging (low rise), relatively more closely or loosely fitted shorts, and other styles, may be provided by varying the ratios listed in Table 1 within (or even beyond) the ranges shown. Moreover, the use of elastomeric or extensible material to form the garment shell may provide additional adaptability to fit a wider range of wearer sizes.

Since the pant dimensions are determined by the dimensions of the intended wearer, the ratios shown are based upon five measurements of an intended wearer, abbreviated as follows:
A: waist circumference (Fig. 1A)
B: hip circumference (Fig. 1A)
C: thigh circumference (measured in crotch region, horizontally; see Fig. 1A)
D: crotch depth (measured in crotch region, viewed 18 inches from the wearer's side; see Fig. 1B)
E: center front waist to center back waist through crotch; see Fig. 1B

Table 2 shows how garment shell dimensions shown in Figs. 5A and 5B are determined using body measurements A-E and ratios in Table 1. Table 2 also shows how the ratios in Table 1 have been applied to create shorts for two different size wearers, one a mannequin of a child (Wearer #1) weighing approximately 32 to 40 pounds (15-18 Kg), the other an adult female (Wearer #2) weighing approximately 125 pounds (57 Kg).

**TABLE 1**

| PANT DIMENSIONS DETAILS and RATIOS EXEMPLARY RANGES | | |
|---|---|---|
| Garment inseam I (Fig. 5A, dimension "T") | Selected based on garment style, There is not a seam at this location; this is simply the location where an "inseam" measurement is generally taken. After contraction, this dimension "T" provides the "hanging legs" feature of the pant. | 1-5 inches (2·5-12·7cm) or more |
| Width of garment shell (Fig. 5A, dimension "W") | Ratio of 2x Width (i.e., garment circumference) to the larger of wearer's Hip or Waist circumference 2w : [B or A] | From about 1.2 : 1 to about 2 : 1, such as about 1.7, e.g. 2w = 1.2A or 1.2B |
| Length of base of are (Fig. 5A, dimension "b") | Ratio of Arc base length to Wearer crotch depth b:D | From about 1 : 1 to about 1.5 : 1, such as about 1.25:1 |
| Circumference of leg opening (Fig. 5A, dimension "c") | Ratio of Leg opening to Wearer thigh circumference c:C | From about 1.1 : 1 to about 1.5 : 1, such as about 1.25:1 |
| Takeup (shortening) of garment shell on gathering of crotch (Fig. 5B, dimension "s") | Ratio of Takeup to 2x Garment inseam length I s : 2I | From about 1 : 1 to about 1.6 :1, such as about 1.3:1 |
| Length of garment shell after gathering (Fig. 5B, dimension "I") | Ratio of Length after gathering to Wearer F to B waist thru crotch 1 : E | This can vary widely depending on the desired short style, but for a standard fit, from about 1.1 : 1 to about 1.4 : 1, such as about 1.25:1, e.g. 1 = 1.4E |
| Length of garment shell before gathering (Fig. 5A, dimension "L") | Sum of Takeup and Length of shell after gathering s + 1 | |
| Arc height (Fig. 5A, dimension "a") | (Width of garment shell - 2x Garment inseam I)/2 (W-2I) /2 | |

**TABLE 2**

| | Wearer #1 | Short #1 | Wearer #2 | Short #2 |
|---|---|---|---|---|
| A | 50 cm | | 78 cm | |
| B | 54 cm | | 96 cm | |
| C | 29 cm | | 55 cm | |
| D | 10 cm | | 16.5 cm | |
| E | 41 cm | | 61 cm | |
| I | | 6 cm | | 8 cm |
| W | | 45 cm | | 67 cm |
| b | | 12.5 cm | | 20.5 cm |
| c | | 36 cm | | 68 cm |
| s | | 15.5 cm | | 21 cm |
| l | | 50.5 cm | | 75 cm |
| L | | 66 cm | | 96 cm |
| a | | 15 cm | | 25 cm |

The pant 10 can also include an absorbent structure 60. The absorbent structure 60 can be attached to the garment shell 64 at the front waist edge 38 and back waist edge 39, or at some point below the front waist edge 38 and back waist edge 39 on the front region 22 and back region 24. (Figs. 2A and 2B). Alternatively, the absorbent structure 60 can be attached to the garment shell 64 in the contracted crotch region 26. The absorbent structure 60 may be either permanently attached to the garment shell 64 or refastenably attached to the garment shell 64 to allow for replacement of absorbent structures 60 when the absorbent structures 60 become soiled.

The absorbent structure 60 can be any structure that is generally compressible, conformable, non-irritating to the skin, and capable of absorbing and retaining liquids and certain body wastes. The absorbent structure 60 can be manufactured in a wide variety of sizes and shapes, from a wide variety of liquid absorbent materials commonly used in the art, and may be stretchable, non-stretchable, or elastic. For example, the absorbent structure 60 can suitably include a matrix of hydrophilic fibers, such as a web of cellulosic fluff, mixed with particles of a high-absorbency material commonly known as superabsorbent material. In a particular embodiment, the absorbent structure 60 includes a matrix of cellulosic fluff, such as wood pulp fluff, and superabsorbent hydrogel-forming particles. The wood pulp fluff can be exchanged with synthetic, polymeric, meltblown fibers or short cut homofil bicomponent synthetic fibers and natural fibers. The superabsorbent particles can be substantially homogeneously mixed with the hydrophilic fibers or can be nonuniformly mixed. The fluff and superabsorbent particles can also be selectively placed into desired zones of the absorbent structure 60 to better contain and absorb body exudates. The concentration of the superabsorbent particles can also vary through the thickness of the absorbent structure 60. Alternatively, the absorbent structure 60 can include a laminate of fibrous webs and superabsorbent material or other suitable means of maintaining a superabsorbent material in a localized area.

Suitable superabsorbent materials can be selected from natural, synthetic, and modified natural polymers and materials. The superabsorbent materials can be inorganic materials, such as silica gels, or organic compounds, such as crosslinked polymers, for example, sodium neutralized polyacrylic acid. Suitable superabsorbent materials are available from various commercial vendors, such as Dow Chemical Company located in Midland, Michigan, U.S.A., and Stockhausen, Inc. in Greensboro, North Carolina, U.S.A. Typically, a superabsorbent material is capable of absorbing at least about 15 times its weight in water, and desirably is capable of absorbing more than about 25 times its weight in water.

In one embodiment, the absorbent structure 60 includes a blend of wood pulp fluff and superabsorbent material. One preferred type of pulp is identified with the trade designation CR1654, available from U.S. Alliance, Childersburg, Alabama, U.S.A., and is a bleached, highly absorbent sulfate wood pulp containing primarily soft wood fibers and about 16 percent hardwood fibers. As a general rule, the superabsorbent material is present in the absorbent structure 60 in an amount of from 0 to about 90 weight percent based on total weight of the absorbent assembly. The absorbent structure 60 suitably has a density within the range of about 0.10 to about 0.35 grams per cubic centimeter. The absorbent structure 60 may or may not be wrapped or encompassed by a suitable tissue or nonwoven wrap that may help maintain the integrity and/or shape of the absorbent assembly.

The absorbent structure 60 can also incorporate other materials that are designed primarily to receive, temporarily store, and/or transport liquid along the mutually facing surface with absorbent structure 60, thereby maximizing the absorbent capacity of the absorbent assembly. One suitable material is referred to as a surge layer (not shown) and includes a material having a basis weight of about 50 to about 120 grams per square meter, and including a through-air-bonded-carded web of a homogenous blend of 60 percent 3 denier (3 g per 9,000 metres) type T-256 bicomponent fiber including a polyester core/polyethylene sheath and 40 percent 6 denier type T-295 polyester fiber, both commercially available from Kosa Corporation of Salisbury, North Carolina, U.S.A.

In particular embodiments, the absorbent structure 60 is thin to provide a slim, comfortable, non-bulky pant 10. Any suitable thin absorbent structure may be used, such as for example, the thin absorbent described in WO 02/49565, published June 27, 2002, by Sawyer et al.

The absorbent structure 60 can include a pair of containment flaps 62 (Fig-3A) which are configured to provide a barrier to the transverse flow of body exudates. A. flap elastic member (not shown) can be operatively joined with each containment flap 62 in any suitable manner as is well known in the art. The elasticized containment flaps 62 define an unattached edge which assumes an upright, generally perpendicular configuration to form a seal against the wearer's body. Suitable constructions and arrangements for the containment flaps 62 are generally well known to those skilled in the art and are described in U.S. Patent 4,704,116 issued November 3, 1987, to Enloe.

As an alternative, a pant-like garment insert could be used for the absorbent structure 60. For example, the pant-like garment insert may include a body side liner, an outer cover, and an absorbent assembly between the body side liner and the outer cover, and side panels. Examples of suitable pant-like garment inserts include a training pant, such as HUGGIBS® PULL-UPS® Disposable Training Pants, or a disposable underpant, such as GOODNITES® Disposable Underpants, both manufactured by Kimberly Clark Corporation, Neenah, Wisconsin, U.S.A. A training pant serving as the pant-like garment insert for the absorbent structure 60 can include front side panels 34 and back side panels 134 (Figs. 2B and 3B). The manufacture of training pants having side panels can be accomplished in the manner described in U.S. Patent No. 6,562,167, issued 13 May 2003 to Coenen et al.

As another alternative, a pad-type absorbent could be used for the absorbent structure. The pad-type absorbent can be attached in the crotch region 26 of the pant 10. An example of a suitable pad-type absorbent is a feminine care pad such as KOTEX® Feminine Napkins, KOTEX® LIGHTDAYS® disposable panty liners, or an incontinence absorbent pad such as POISE® Feminine Guards and Pads or DEPEND® Guards for Men, all manufactured by Kimberly-Clark Corporation, Neenah, Wisconsin, U.S.A.

For reference, arrows 48 and 49 depicting the orientation of the longitudinal axis and the transverse axis, respectively, of the garment shell 64 are illustrated in Figs. 3A, 3C, and 5.

The garment shell 64 is suitably constructed of materials that are comfortable against the skin and non-irritating. It is contemplated that the garment shell 64 can be either disposable or durable. Both nonwoven and woven materials are contemplated for the garment shell 64. For example, the garment shell 64 for pant 10 can be selected from a wide variety of materials, including elastic, stretchable, or nonstretchable materials. The garment shell 64 can be a single layer of material or a multi-layered laminate structure. One example of a suitable material is a spunbond polypropylene nonwoven web. The garment shell 64 itself may be absorbent and, for example, may be made of those materials of which the absorbent structure 60 is made. For instance, the garment shell 64 may include a coform material with a polyethylene film on an outer surface of the garment. The garment shell 64 suitably provides a relatively cloth-like texture to the wearer.

The present invention also includes various methods for making pants from a web. Referring to Fig. 4, a single web 100 is provided moving in the direction represented by arrow 102. Alternatively, two webs that are joined at their edges to form a double-width piece (not shown) can be used for the web 100. The web 100 may be a flat web and can be composed of any material previously described for the garment shell 64.

The method can be carried out using machine direction assembly so that arrow 102 can correspond to the longitudinal direction parallel to the longitudinal axis 48 as shown in Fig. 5 with the products connected end-to-end or waist-to-waist, or the method can be carried out using cross direction assembly so that arrow 102 can correspond to the transverse direction parallel to the transverse axis 49 as shown in Fig. 10A with the products connected side-to-side.

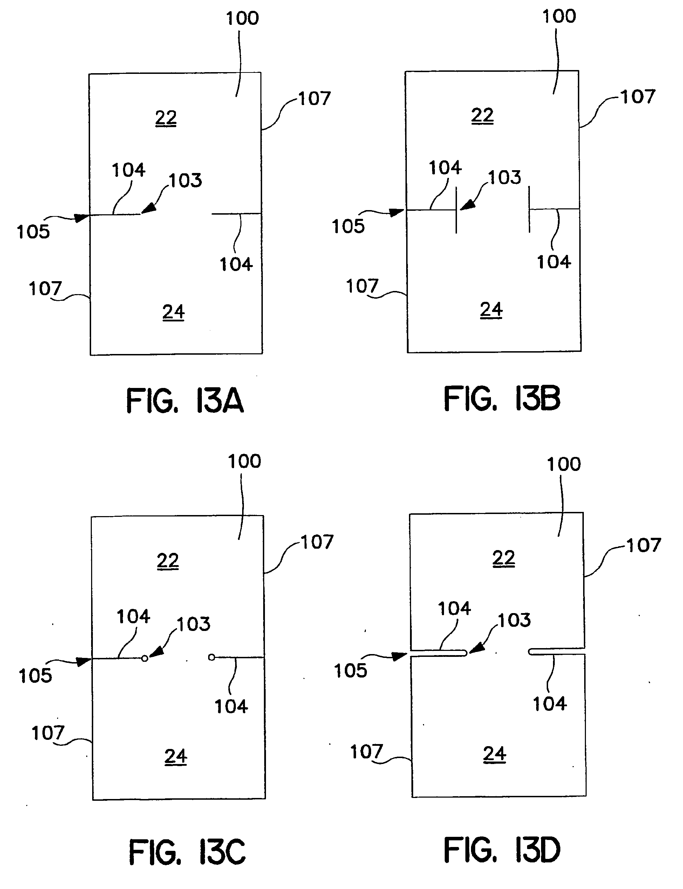

In both the machine direction process (Figs. 5-9) and the cross direction process (Figs. 10A-12), the web 100 is cut along each of the transversely opposed edges 107 of the web 100 to define leg openings 104 (Figs. 5 and 10A). More particularly, the leg opening 104 is formed by slitting the web 100. The geometry of the leg opening 104 affects the overall product appearance. Examples of suitable cuts for creating leg openings 104 are illustrated in Figs. 13A-13C

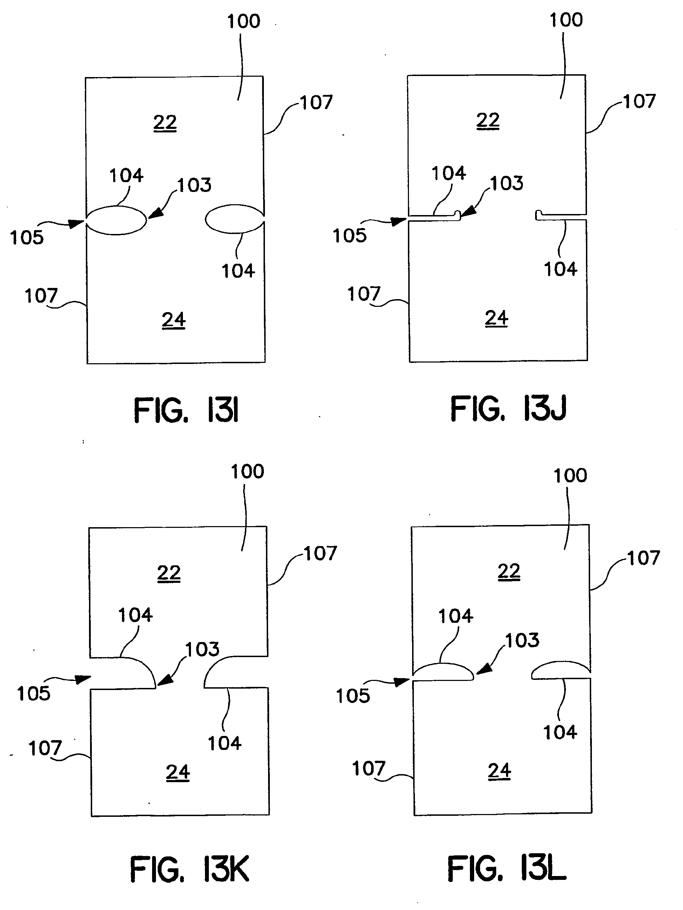

When in a flat configuration, as illustrated in Figs. 13A-13L, the leg openings 104 are slits (Figs 13A-C) within the web. Also shown, but not within the scope of the present invention, are symmetrical (Figs. 13D-I) or asymmetrical (Figs. 13J-L) portions cut and removed from along each of the transversely opposed edges 107 of the web. Any suitable symmetrical or asymmetrical shape may be cut to form the leg openings 104. As referred to herein, the symmetry of the leg opening cut-outs is determined with respect to a transverse axis 49 through the web. Alternatively, the leg openings 104 may be formed by folding material adjacent to a slit in order to move a portion of the material out of the way.

As illustrated in Fig. 13A, the leg openings 104 may be formed from single slits. Slits can result in longer legs in the garment compared to leg openings created from portions of the web that are cut out and removed from the remainder of the web. Alternatively, the leg openings 104 may be formed from T-shaped slits, as shown in Fig. 13B. Expanding the interior end 103 of the slits into a "T" shape provides pant legs that hang smoothly adjacent to the crotch region 26. Additionally, the portion of the slit extending from the interior end 103 to an open end 105 of the T-shaped slit may be hemmed along one or both edges forming this portion of the slit. Similarly, in embodiments other than T-shaped slits, a portion of the web adjacent to the cut may be folded and manipulated out of the way to create a larger leg opening 104.

Slits may be cut using pinch-cut knives, intermittent slitters, or any other suitable straight machine-direction or cross-direction cut. Not only do the slits result in longer legs on the garment, but less web 100 material waste accrues than in the cut-out embodiments. The slits may be reinforced or otherwise adapted at the shaped interior ends 103 of the leg openings, as shown in Fig. 13C and described in further detail below. As another alternative, the slits need not initially extend all the way to the transverse edges 107 of the web, but instead may be cut within the web for easier handling of the web during the pant-forming process, and cut at the transverse edges 107 of the web later during the pant-forming process.

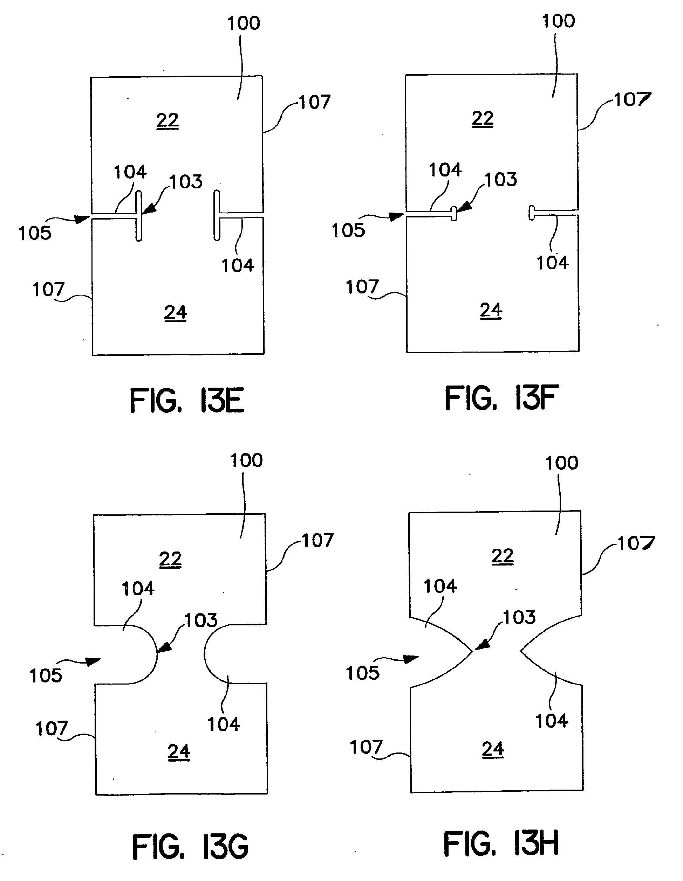

In an alternative method not within the scope of the present rather than slits, the leg openings 104 are formed from slots, which as used herein refers to cut-outs that resemble the shape of slits but with at least some portion of the web 100 removed from the remainder of the web. The slots may be symmetrical, as illustrated in Figs. 13D-F, or asymmetrical, as illustrated in Fig. 13J. More particularly, the slots may form substantially straight lines, as shown in Figs. 13D and 13J, or T-shaped slots, as shown in Fig. 13E, or a slot having a reinforced interior end 103 resembling a hairpin shape, as shown in Fig. 13F.

In other methods outside the scope of present invention, other suitable symmetrical shapes mat may be cut and removed from the web 100 to form the leg openings 104 include a "U" shape, as illustrated in Fig. 13G, as well as a "mound" shape, as illustrated in Fig. 13H. The U-shaped leg opening 104 results in relatively short garments legs, whereas mound-shaped leg openings 104 may provide more body coverage than the U-shaped leg openings 104. The term "mound-shaped" refers to a cut-out portion having an angle at the interior end 103 that is less than 180 degrees, thereby resulting in a leg opening 104 having a triangular shape, or a softened triangular shape that may resemble the shape of a mound or a mountain.

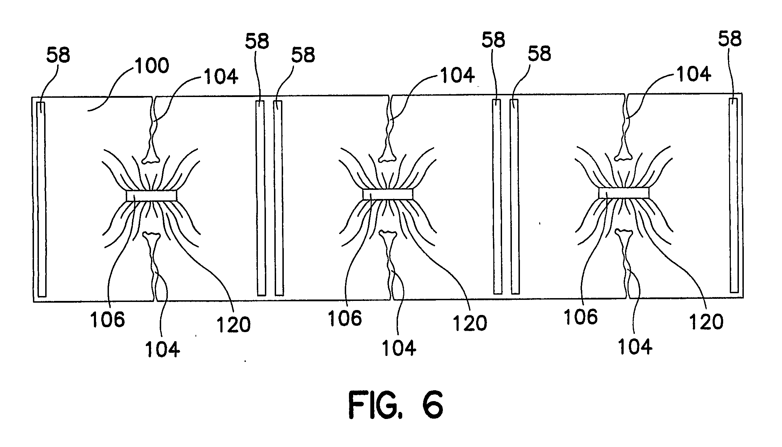

Rather than expanding from the interior end 103 of the leg opening 104 to the open end 105 of the leg opening, the leg openings 104 may be tapered at the open ends 105, thereby resulting in a teardrop shape. The tapered shape can provide a straight horizontal appearance along the leg ends of the garment even though the contracted area 120 (as shown in Figs. 6 and 11) distorts the lower region of the garment. The tapered shape may be either symmetrical, as illustrated in Fig. 13 I, or asymmetrical, as illustrated in Fig. 13L.

As an alternative to slits and/or symmetrical cut-outs, in a method outside the scope of the present invention, leg openings 104 may be any suitable asymmetrical shape. For example, as shown in Fig. 13K, the leg openings 104 may include a straight edge along a front edge of the cut-out and a curvilinear edge along a back edge of the cut-out. This asymmetrical design may provide greater butt coverage in the back of the garment and longer legs in the front of the garment.

Many of the shapes of the leg openings 104 may be adapted for reinforcement by cutting a circular cut-out at the interior end 103 of the leg openings 104 to reduce stress concentration at the interior end of the openings, thereby reducing the likelihood of tearing in the crotch region 26. An example of this type of reinforcing cut-out is illustrated in Fig. 13C. The reinforcing cut-out may be other suitable shapes besides circular. For example, when the leg openings 104 are formed from slots, the reinforcing cut-out may have a shape that is wider than the longitudinal opening of the slot and narrower than the transverse opening of the slot to reduce the stress concentration. A suitable shape may be circular or oblong, as illustrated in Fig. 13F.

As more fully described below, the leg openings 104 become the leg openings 52 of the pant 10.

In the machine direction process (Figs. 5 and 6-9), strips 106 may be applied to selected areas located between the leg openings 104. Strips 106 can include elastic for non-elastic materials Examples of suitable non-elastic material include heat contractible materials, such as heat shrinkable films, for example, films formed of polyether block amides (PEBAX®, available from the Atofina Company of France) or the like. If the strips 106 are elastic, the elastic can be formed of any suitable material previously described for the waist elastic member 58. As an alternative, strips 106 can include any of the previously described delayed retraction materials.

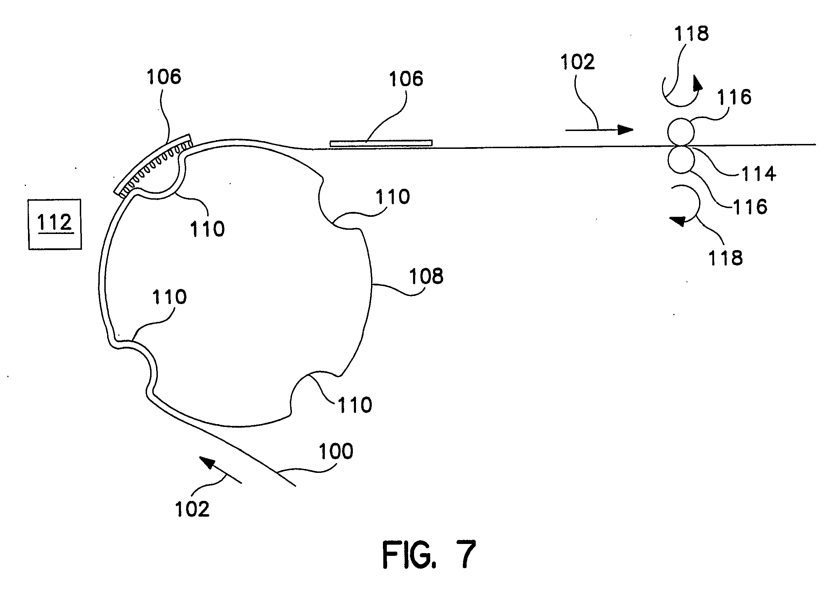

Referring to Fig. 7, if the strips 106 are elastic, the strips 106 can be applied to the web 100 using a looper drum 108. Looper drums like looper drum 108 are known and are described, for example, in U.S. Patent 5,171,388 issued December 15, 1992 to Hoffman et al. Drum 108 includes surface grooves 110. Drum 108, as illustrated in Fig. 7, includes four surface grooves 110, but any number of surface grooves 110 may be included. The surface grooves 110 are spaced around the drum 108 so that each garment shell 64 eventually includes one strip 106. The web 100 travels around the drum 108 in the direction of arrow 102. The web 100 runs down into the surface grooves 110 by virtue of the fact that the drum 108 includes apertures across its surface and is under vacuum. Adhesive (shown for purposes of illustration as dots between strip 106 and the web 100 over the surface groove 110) can be applied to the strip 106. Alternatively, the adhesive can be applied to the web 100 in the selected areas between leg openings 104. Suitable adhesives, which can be applied continuously or intermittently as beads, a spray, parallel swirls, or the like, can be obtained from Bostik Findley Adhesives, Inc., of Wauwatosa, Wisconsin, U.S.A., or from National Starch and Chemical Company, Bridgewater, New Jersey U.S.A.

The web 100 passes by the elastic application module 112 and the strip 106 of elastic is applied in a substantially unstretched condition to the web 100 over the surface groove 110. The web 100 with the strip 106 of elastic continues moving in the direction of arrow 102 out of surface groove 110 and off the drum 108. The web 100 with strip 106 of elastic passes through nip 114 to press and secure the strip 106 of elastic to the web 100. The nip 114 is defined by rolls 116 turning in the direction of arrows 118. In the alternative, any other suitable method for pressing and securing the strip 106 of elastic to the web 100 can be used. As web 100 exits the nip 114, the web 100 can be drawn at a slower rate by the downstream process than the surface speed of rolls 116, allowing the strip 106 of elastic to contract and reduce the length of web 100.

Fig. 6 shows the web 100 after the contraction of the strips 106. The contraction of the web 100 defines contracted areas 120 in the selected areas between leg openings 104. The contracted area 120, as described more fully below, becomes the contracted crotch region 26 of the pant 10.

Alternatively, the strip 106 can be applied to the web 100 by any other method known in the art such as, for example, a corrugating drum such as that described in U.S. Patent 4,397,704 issued August 9, 1983 to Frick, or an elastic application system in which the material is gathered into folds running in the cross direction and a continuous elastic is applied in the machine direction and severed at the location of the folds in the base material such as described in U.S. Patent 4,417,938 issued November 29, 1983 to Sigl, or an intermittent adhesive application that allows the elastic to snap back from non-adhesive zones, a high efficiency interface roll such as that described in U.S. Patent 6,022,443 issued February 8, 2000 to Rajala et al., U.S. Patent 5,556,504 issued September 17, 1996 to Rajala et al., and U.S. Patent 6,319,347 issued November 20, 2001 to Rajala et al., or by any other any means known in the art.

Figs. 6 and 11 also show waist elastics 58 applied to the web 100. The waist elastics 58 can be applied by any method known in the art at any stage in the manufacturing of the pant 10.

As an alternative, the tension on the web 100 can be reduced by cutting the web 100 into separate pieces approximately midway between successive strips 106 to define a garment shell 64 (Fig. 3C). It is also contemplated, however, that the step of cutting the web 100 can be carried out after contraction of the web 100. It is further contemplated that, instead of a continuous web of multiple garment assemblies connected to one another, the web 100 may exist as a single garment assembly or garment shell 64 at the outset of the process. This option exists in both the machine direction process as well as the cross direction process.

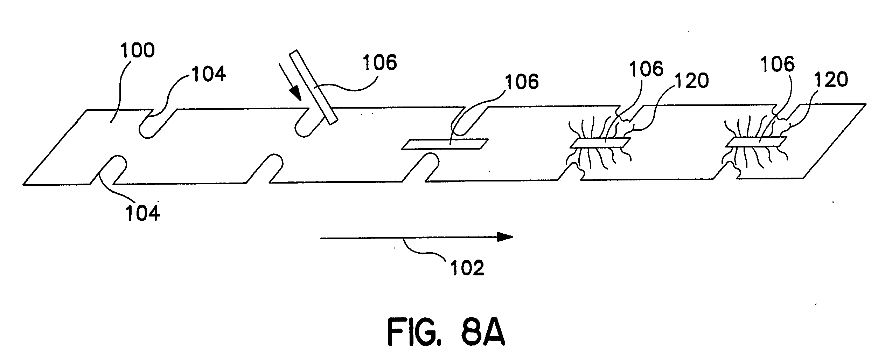

Referring to Fig. 8A, the strips 106, whether elastic or nonelastic, can be applied to the selected areas of the web 100 between the leg openings 104 by a cut-and-place module (not shown) as is commonly known in the art.

Next, the web 100 can be contracted elastically or inelastically by any suitable means. For example, if the strip 106 is an elastic capable of delayed retraction, the web 100 can be contracted by activating the strip 106 to restore the elasticity by time, temperature, radiation or other appropriate energy. If the strip 106 is a heat shrinkable material, the web 100 can be contracted inelastically by activating the heat shrinkable material by applying heat or other appropriate energy.

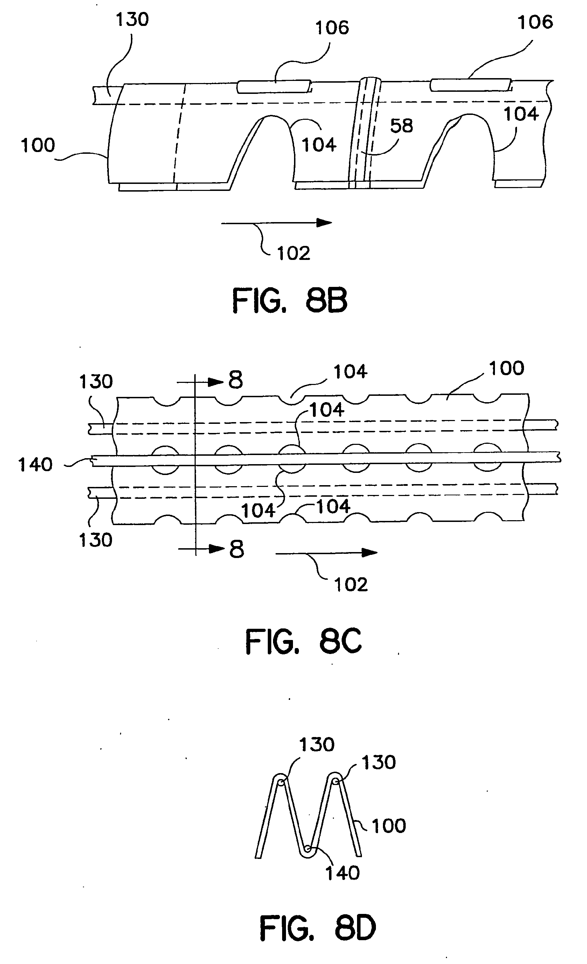

In certain embodiments, the web 100 may be folded against a support structure 130 (Figs. 8B and 8D). Examples of suitable support structures include internal support structures such as bars over which the web may be folded, or external support structures such as opposing vacuum conveyors between which the web may be folded. This folding may occur any time prior to the final product cutoff. When the process is being carried out in the machine direction, as shown in Fig. 8B, the web 100 is folded along its longitudinal centerline. The contracted crotch region 120 may be formed against the fold while the web 100 is positioned on the support structure 130. When the contraction involves application of a strip 106 or other additional piece of material, the support structure 130 within the folded area of the web 100 may provide useful opposition to the application of the strip. In particular, if the strip 106 is pre-stretched it may be helpful to have an object such as the support structure 130 against which to stretch the strip during or prior to application. Alternatively, the strip 106 may be applied to the web 100 when positioned between the web 100 and the support structure 130. Additionally, when the web 100 is folded along the longitudinal centerline, both leg openings 104 may be cut simultaneously with a single cutting action. An absorbent structure 60, if desired, can be attached to the web 100 before the web is folded, while the web is folded, or after unfolding the web. For instance, the fold of the web 100 may be inverted around an absorbent structure 60, such that a strip 106 may be applied to an inner surface of the web 100 while in a convex position over the support structure 130, or an absorbent structure 60 may be applied to the web 100 over the strip 106 after removing the web 100 from the support structure 130 and inverting the web 100 into a concave position into which the absorbent structure 60 may be inserted.

The process may also be carried out using a multi-lane production system for even greater efficiency, as illustrated in Fig. 8C. A cross-section of the multi-lane set-up is illustrated in Fig. 8D. When using the multi-lane set-up, each pant assembly is folded against a single support structure 130. The pant assemblies on a single support structure 130 are connected end-to-end or waist-to-waist, and each pant assembly is connected along at least one transverse edge 107 to another pant assembly on an adjacent support structure 130. Transversely adjacent pant assemblies may be slit apart or otherwise separated at point 140 in Fig. 8D, for example, at any suitable point during the process. For example, the support structure 130 may include upper and lower support bars, in which case point 140 may be a lower support bar, and the lane slitting may occur at point 140 or between the upper and lower support bars 130, 140; in either case, the slitting occurs along adjoining edges 107 to separate adjacent assemblies.

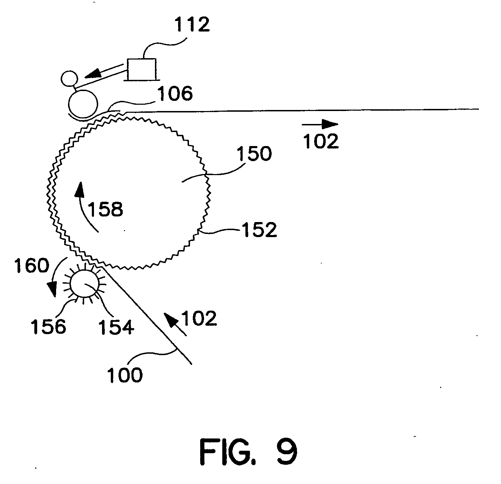

In particular embodiments, the strips 106 may be applied to the web 100 after contraction or pregathering of the web 100. The application of the strips 106 need not necessarily take place in conjunction with the folding process. In the machine direction, the web 100 can be pregathered by corrugating in the selected areas between the leg openings 104 by using a corrugating drum 150 (Fig. 9) in preparation for attachment of strip 106. Corrugating drums like corrugating drum 150 are known and are described, for example, in previously mentioned U.S. Patent 4,397,704 issued August 9, 1983 to Frick. Alternatively, a drum with discontinuous grooves that correlate with the location of strips 106 can be used. The web 100 travels around the drum 150 in the direction of arrow 158. Pressing roll 154 has teeth 156. The web 100 is pushed down into the grooves 152 by the teeth 156, thereby corrugating the web 100. Drum 150 and pressing roller 154 move in the direction of arrows 158 and 160, respectively.

Next, the strips 106 can be applied to the corrugated web 100 by a conventional cut-and-place applicator or other appropriate apparatus. Strips 106 can be attached to the web 100 using adhesive, thermal or ultrasonic bonding, or other means known in the art. Use of a corrugating drum or other device to pregather the web 100 permits the use of an unstretched elastic or of a non-elastic, non-retractive material such as a film or nonwoven material with properties similar to the web 100. Alternatively, the strip 106 may include any of the previously described materials. The strips 106 maintain the corrugation in the contracted area 120 (Fig. 6).

In the cross direction process (Figs. 10A-12), as in the machine direction process, strips 106 can be applied to the selected areas located between the leg openings 104. In the cross direction assembly process, strips may be applied on the web 100 in an orientation essentially parallel with the longitudinal axis 48, as shown in Fig. 10A.

The application of strip 106 of elastic material can be accomplished by a variety of methods, such as by moving the distal edges of the web 100 closer together and allowing the center portion of the web to become looped using the same principles of the previously described looper drum, but with the strip 106 being applied in an orientation perpendicular to arrow 102, or by other methods as are known in the art. As with the previously described looper drum, the web 100 can be fully extended again after application of the strip 106 in order to fully adhere the strip 106 to the web 100. In alternative embodiments, the strips 106 can be applied to the web 100 by a process in which an elastic or inelastic piece of material is cut, rotated and placed onto the web 100, for example, as described in U.S. Patent 5,716,478 issued February 10, 1998 to Boothe et al., U.S. Patent 5,759,340 issued June 2, 1998 to Boothe et al. and U.S. Patent 4,608,115 issued August 26, 1986 to Schroth et al., or by any other means known in the art. Where the strip 106 is a heat contractible material or a material capable of delayed retraction, the strip can be applied to web 100 as the web travels in the direction of arrow 102 (Fig. 10A) in a flat and unlooped state.

The web 100 can be contracted elastically or inelastically by any of the previously described methods. Fig. 11 shows the web 100 after the contraction of the strips 106. The contraction of the web 100 defines contracted area 120 in the selected areas between the leg openings 104. The contracted area 120, as described more fully below, becomes the contracted crotch region 26 of the pant 10.

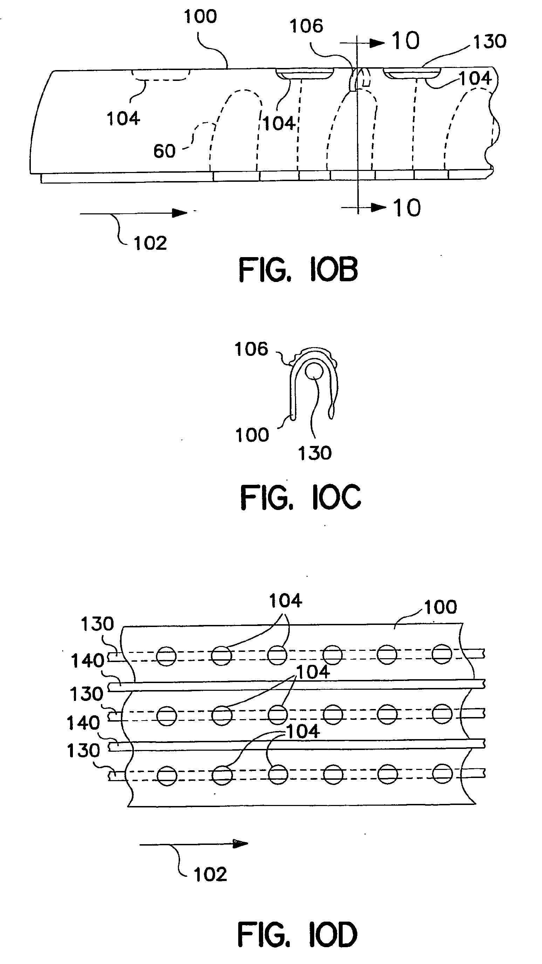

As described above, the web 100 may be folded against a support structure 130 in certain embodiments. This folding may occur any time prior to the final product cutoff. When the process is being carried out in the cross direction, as shown in Fig. 10B, the web 100 is folded perpendicular to the longitudinal centerlines of the individual garments within the web 100. The contracted crotch region may be formed while the web 100 is folded and positioned on the support structure 130, or before or after the web 100 is positioned on the support structure 130. When the contraction involves application of a strip 106 or other additional piece of material, the support structure 130 against the folded web 100 may provide useful opposition to the application of the strip 106. In particular, if the strip 106 is pre-stretched it may be helpful to have an object such as the support structure 130 against which to stretch the strip during application. In certain embodiments, such as when the support structure 130 includes opposing vacuum conveyors, two or more separate strips 106 may be applied to the web 100 on opposite sides of the fold. As explained above with respect to the machine direction process, the strip 106 may, alternatively, be positioned between the web 100 and the support structure 130 when applying the strip 106 to the web 100.

Additionally, when the web 100 is folded perpendicular to the longitudinal centerline or longitudinal axis 48 of the garment assemblies, the leg openings 104 may be cut while the web 100 is on the support structure 130. An absorbent structure 60, if desired, can be attached to the web 100 before the web is folded, while the web is folded, or after unfolding the web. Alternatively, as described above with respect to the machine direction process, the web 100 may be inverted prior to attaching the absorbent structure 60 to the web 100. This folded configuration of the web 100 may facilitate easier insertion of the absorbent structure 60 since the length from the front waist edge 38 to the back waist edge 39 through the crotch region 26 may differ between the absorbent structure 60 and the garment shell 64, particularly before the crotch region 26 is contracted. Such a difference may be better accommodated when both components are folded, thus bringing the waist edges 38, 39 into close proximity to one another. As illustrated in Fig. 10C, the absorbent structure 60 need not be stretched to fit the garment shell 64, nor does the garment shell 64 have to be gathered to fit the absorbent structure, as may be required in a flat process.

As also described above with respect to the machine direction conveyance of the web 100, the process may also be carried out using a multi-lane production system in the cross direction conveyance, as illustrated in Fig. 10D. When using the multi-lane set-up in the cross direction, each machine-direction array of pant assemblies is folded against a single support structure 130. The pant assemblies on a single support structure 130 are connected side-to-side, and each pant assembly is connected waist-to-waist to another pant assembly on an adjacent support structure 130. The slitting apart of transversely adjacent pant assemblies may occur along the fold line (if any) between the waists of adjacent garment assemblies at any suitable point during the process.

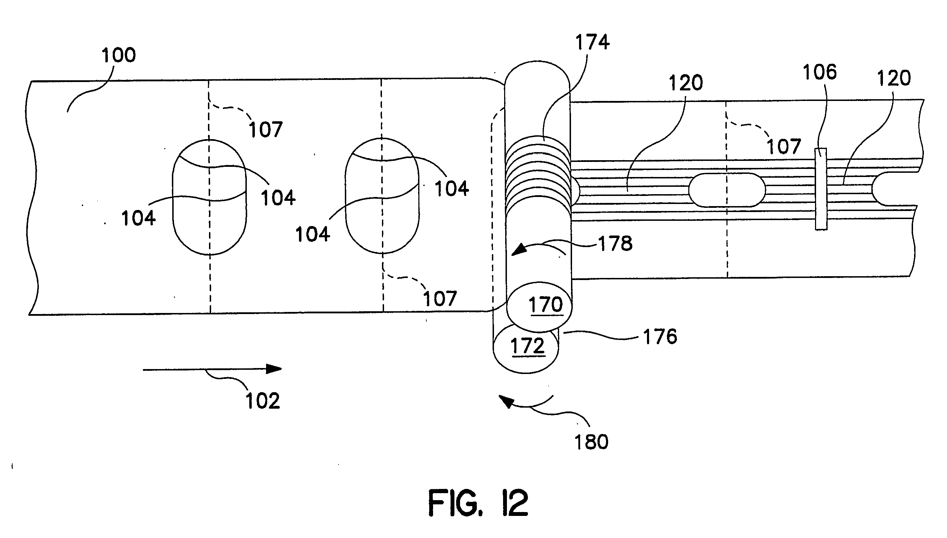

In particular embodiments, the strips 106 are applied to the web 100 after contraction or pregathering of the web 100. In the cross direction, the web 100 can be pregathered by corrugating in the selected areas between the leg openings 104 by using intermeshing grooved rollers 170 and 172 (Fig. 12) in preparation for attachment of strip 106. Intermeshing grooved rollers like 170 and 172 are known in the art and are described, for example, in U.S. Patent 5,755,902 issued 26 May 1998 to Reynolds. Roller 170 includes grooves 174 only in the middle portion of the roll to correspond to the desired location of the contracted area 120 on the web. The web 100 travels through nip 176 formed by rolls 170 and 172 in the direction of arrow 102. Roller 172 has complementary grooves (not shown) designed to intermesh with grooves 174 of roller 170. The web 100 is pushed into the grooves 174 by the complementary grooves on roll 172 to provide the corrugation in the contracted area 120. Rolls 170 and 172 move in the direction of arrows 178 and 180, respectively. The corrugations are held in place by attaching strips 106 on top of the corrugations.

The strip 106 can be applied to the corrugated web 100 by a cut-and place module, or similar technology, as is commonly known in the art and can be attached to the web using thermal, ultrasonic or adhesive bonding, or any other means known in the art. The strip 106 may include an inextensible material such as a film or nonwoven material with properties similar to web 100, or may include any of the previously described materials.

In either the machine direction process or the cross direction process, the web 100 can now be cut into individual pieces, each of which will form a garment shell 64. The cutting can be accomplished by, for example, pinch cutting, shear cutting, or any other means known in the art. As another alternative, the web 100 can be provided as separate pre-cut pieces each of which pre-cut separate pieces will eventually become a single garment shell 64, so that this cutting step could be skipped and the process could start with a pre-cut piece as the web 100. Fig. 3C shows the garment shell 64 prior to folding and formation of the side seams 54. As shown and as previously mentioned with respect to Figs. 1, 2A, and 2B the garment shell 64 can include a front region 22, a back region 24, a contracted crotch region 26, an inner surface 28, and an outer surface 30 (not shown), front waist edge 38, back waist edge 39, and waist elastic member 58. The garment shell 64 can also include strip 106. It is also contemplated that the garment shell 64 can be made upside-down, i.e., with the inner surface 28 facing downwardly (not shown). The garment shell 64 can then be folded and the side seams 54 formed by any conventional method known in the art to form the pant 10 (without an absorbent structure). It is contemplated that the step of contracting the web 100 can occur either before or after the step of cutting into individual garment shells 64, and also before or after the formation of the side seams 54.

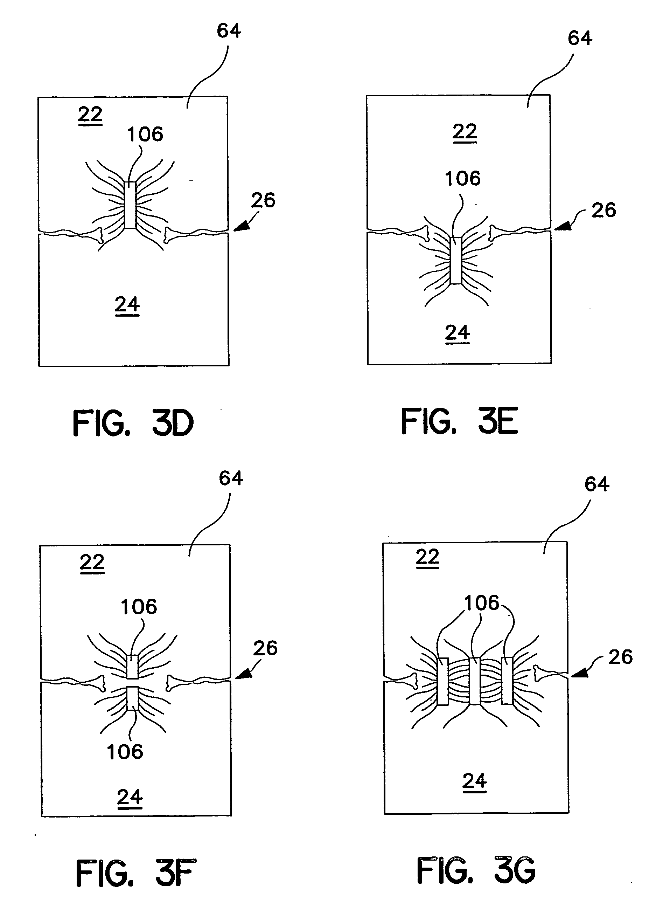

In either the machine direction process or the cross direction process, in alternative embodiments, the strip 106 need not be a single strip of material. In particular embodiments, elastic strands or ribbons as are known in the art can be used instead of a single strip of material for strip 106. The elastic strands or ribbons can be straight or curved. Alternatively, the contracted crotch region 26 may include one or more strips 106 longitudinally offset, such as shown in Figs. 3D and 3E, or multiple strips 106 arranged in a segmented manner, either spaced apart longitudinally (Fig. 3F) or spaced apart transversely (Fig. 3G). In certain embodiments, the strip may be, at most, one-third the length of the garment shell when the garment shell is in a laid-flat, fully extended, namely uncontracted, condition. In addition, in the embodiments in which the web is corrugated or otherwise gathered, it is contemplated that instead of attaching a strip 106, the corrugation or gathers in the contracted area 120 can be maintained by fusing or bonding the corrugations together in the selected areas between the leg openings 104. The corrugations can be bonded to themselves to hold them in place by adhesive, thermal, or pressure bonding, or by any other means known in the art.

In the machine direction process, the strip 106 need not be a separate piece of material applied to the web 100. Instead, the web 100 may include an integral elastic zone aligned along the machine direction center line, instead of strip 106, with the elastic zone active in only the crotch region. Elasticization of only the crotch region of the pant may be achieved by, for example, an elastic laminate structure in which the elastic is attached to the laminate using an intermittent adhesive. Intermittent adhesive application would allow the elastic to snap back from non-adhesive zones, which would be uncontracted as a result; contracted, adhesive-bearing zones can be located only in the crotch region of the garment. As an alternative, the elastic nature of certain regions may be inactivated by chopping or overbonding the elastic or other methods known in the art, for example, as described in U.S. Patent 6,248,097 issued June 19, 2001 to Beitz.

Referring to Figs. 2A, 2B, 3A, and 3B in particular embodiments, an absorbent structure 60 is included in the pant 10. The absorbent structure 60 can be introduced into the pant 10 in any suitable manner known in the art. In particular embodiments, the absorbent structure 60 can be placed on top of the contracted crotch region 26 on the inner surface 28 of the garment shell 64, either prior to formation of side seams 54 or after side seams 54 are made. It is also contemplated, however, that the absorbent structure 60 can be attached prior to contracting and/or cutting the web 100. Where the absorbent structure 60 is added to the pant 10 prior to formation of side seams 54, cut and place methods such as are known in the art may be used. Alternatively, for a closed pant (i.e., side seams already formed), the absorbent structure 60 may be inserted into the pant such as by the method described in the PCT Publication WO 02/52967 by Rabe, et al., or by other means as may be known in the art. The absorbent structure 60 can be attached to the garment shell 64 at the front waist edge 38 and back waist edge 39, or at some point below the front waist edge 38 and back waist edge 39 on the front region 22 and back region 24. Additionally or alternatively, the absorbent structure 60 can be attached in the contracted crotch region 26. The attachment can be accomplished by ultrasonic or adhesive bonding, or any other suitable method known in the art. As shown in Figs. 2A and 2B, attachment to the front and back regions 22 and 24 provides for a loose fit of shell 64 in the contracted crotch region 26, while the absorbent structure 60 is still held close to the body.

In particular embodiments, the absorbent structure 60 is stretchable or elasticizable in order to provide the desired close to the body fit for the absorbent structure 60 while the garment shell 64 hangs loosely. Alternatively, a suspension system for the absorbent structure may be required to provide a loose fit for the garment shell 64, such as described in U.S. Patent 6,168,585 issued January 2, 2001 to Cesco-Cancian.

The garment shell 64 with the absorbent structure 60 can then be folded and the side seams 54 formed by any conventional method known in the art to form the pant 10, as shown in Figs. 2A and 2B. After folding the garment shell 64 and forming the side seams 54 (with or without an absorbent structure 60), if a temporarily inhibited elastic or latent elastic is used as the waist elastic 58, it may need to be activated to restore the elasticity. Alternatively, the elastics may be activated prior to seaming.

The various components of the pant can be connected together by any means known to those skilled in the art such as, for example, adhesive, thermal and/or ultrasonic bonds, pressure bonds and also sewing and other methods used in durable garment manufacturing. Most of the components may be connected using ultrasonic bonding for improved manufacturing efficiency and reduced raw material costs. For example, in particular embodiments, the side seams 54 are made using ultrasonic bonding. Certain garment manufacturing equipment which is readily known and understood in the art, including frames and mounting structures, ultrasonic and adhesive bonding devices, transport conveyors, transfer rolls, guide rolls, tension rolls, and the like, have not been shown in the Figures.

It will be appreciated that details of the foregoing embodiments, given for purposes of illustration, are not to be construed as limiting the scope of this invention. Although only a few exemplary embodiments of this invention have been described in detail above, those skilled in the art will readily appreciate that many modifications are possible in the exemplary embodiments without materially departing from the novel teachings and advantages of this invention. For example, features described in relation to one embodiment may be incorporated into any other embodiment of the invention. Accordingly, all such modifications are intended to be included within the scope of this invention, which is defined in the following claims and all equivalents thereto. Further, it is recognized that many embodiments may be conceived that do not achieve all of the advantages of some embodiments, particularly of the preferred embodiments, yet the absence of a particular advantage shall not be construed to necessarily mean that such an embodiment is outside the scope of the present invention.

## Claims

1. A pant (10) made from a web (100), the pant comprising:
a garment shell (64), the garment shell (64) including a front region (22), a back region (24), a crotch region (26), a front waist edge (38), a back waist edge (39), side seams (54) connecting the front region (22) to the back region (24), two leg openings (52) and hanging legs (23), at least a portion of each of the front region (22), the back region (24), the crotch region (26), and the hanging legs (23) comprising portions of the web (100), wherein the leg openings (52) are formed from cuts (104) along two transversely opposed edges (107) of the web (100) and the hanging legs (23) include no elasticization around a full periphery of the leg openings (52),
**characterised in that**:
the cuts (104) along the transversely opposed edges (107) of the web (100) consist of slits.

2. The pant (10) of claim 1, further comprising a strip (106) in the crotch region (26), wherein the strip (106) is at most one-third the length of the garment shell (64) when the garment shell (64) is in a laid-flat, fully extended condition.

3. The pant (10) of claim 1 or 2, further comprising a strip (106) in the crotch region (26), wherein the strip (106) comprises a delayed retraction material.

4. The pant (10) of any one of the preceding claims, further comprising an absorbent structure (60) attached to the garment shell (64) in at least one of the front region (22), the back region (24) and the crotch region (26).

5. A method of making the pant (10) of any one of the preceding claims, comprising:
providing a web (100) having a first region and a second region;
cutting at least one portion (104) of the web to define two leg openings (52), wherein the at least one cut portion (104) further defines a separation between the first region and the second region;
contracting the web (100) in a selected area (26) to define at least one contracted area between the first region and the second region; and
attaching the first region and the second region together to form side seams (54), wherein said cutting at least one portion (104) of the web comprises slitting said web (100).

6. The method of claim 5, wherein no portion of the web (100) within the cut portion is removed.

7. The method of claim 5, further comprising cutting a cut-out (103) at an interior end of the cut portion (104).

8. The method of any one of claims 5 to 7, comprising folding the web (100) against a support structure (130).

9. The method of claim 8, comprising applying at least one strip (106) to the folded web in the at least one selected area (26).

10. The method of claim 8 or 9, comprising cutting the at least one portion of the web (104) while the web is folded.

11. The method of any one of claims 5 to 10, further comprising attaching an absorbent structure (60) to the web (100).

12. The method of claim 11, comprising attaching the absorbent structure (60) to the web while the web (100) is folded.

13. The method of claim 11, comprising attaching the absorbent structure (60) to the web (100) prior to folding the web (100).

14. The method of claim 11, comprising attaching the absorbent structure (60) to the web (100) after unfolding the web (100).

15. The method of any one of claims 5 to 14, wherein the web (100) comprises a single pant assembly.

16. The method of any one of claims 5 to 14, wherein the web (100) is a continuous web comprising a plurality of pant assemblies connected to one another.

17. The method of claim 16, further comprising folding the web (100) against at least two support structures (130, 140) each parallel to a direction (102) in which the web (100) is conveyed, wherein each machine-direction array of pant assemblies is folded against a single support structure (130, 140).

18. The method of any one of claims 5 to 17, comprising conveying the web (100) in a machine direction assembly process.

19. The method of any one of claims 5 to 17, comprising conveying the web (100) in a cross direction assembly process.

## Patentansprüche

1. Hose (10), welche aus einer Bahn (100) hergestellt ist, wobei die Hose umfasst:
eine Bekleidungshülle (64), wobei die Bekleidungshülle (64) einen Vorderbereich (22), einen Hinterbereich (24), einen Schrittbereich (26), einen vorderen Taillenrand (38), einen hinteren Taillenrand (39), Seitennähte (54), die den Vorderbereich (22) mit dem Hinterbereich (24) verbinden, zwei Beinöffnungen (52) und hängende Beine (23) beinhaltet, wobei zumindest ein Teil von jedem des Vorderbereichs (22), des Hinterbereichs (24), des Schrittbereichs (26) und der hängenden Beine (23) Teile der Bahn (100) umfasst, wobei die Beinöffnungen (52) aus Schnitten (104) entlang von zwei quer gegenüberliegenden Rändern (107) der Bahn (100) gebildet sind, und wobei die hängenden Beine (23) entlang einer kompletten Peripherie der Beinöffnungen (52) keine Elastizität aufweisen,
**dadurch gekennzeichnet, dass**:
die Schnitte (104) entlang der quer gegenüberliegenden Ränder (107) der Bahn (100) aus Schlitzen bestehen.

2. Hose (10) gemäß Anspruch 1, welche des Weiteren einen Streifen (106) in dem Schrittbereich (26) umfasst, wobei der Streifen (106) höchstens ein Drittel der Länge der Bekleidungshülle (64) ausmacht, wenn sich die Bekleidungshülle (64) in einem flach gelegten, komplett ausgedehnten Zustand befindet.

3. Hose (10) gemäß Anspruch 1 oder 2, welche des Weiteren einen Streifen (106) in dem Schrittbereich (26) umfasst, wobei der Streifen (106) ein verzögert zurückziehendes Material umfasst.

4. Hose (10) gemäß einem der vorherigen Ansprüche, welche des Weiteren eine absorptionsfähige Struktur (60) umfasst, die an der Bekleidungshülle (64) in mindestens einem von dem Vorderbereich (22), dem Hinterbereich (24) und dem Schrittbereich (26) befestigt ist.

5. Verfahren zum Herstellen der Hose (10) gemäß einem der vorherigen Ansprüche, welches umfasst:
Bereitstellen einer Bahn (100), welche einen ersten Bereich und einen zweiten Bereich aufweist;
Schneiden mindestens eines Teils (104) der Bahn, um zwei Beinöffnungen (52) zu definieren, wobei der mindestens eine Schnittteil (104) des Weiteren eine Trennung zwischen dem ersten Bereich und dem zweiten Bereich definiert;
Zusammenziehen der Bahn (100) in einem ausgewählten Bereich (26), um mindestens einen zusammengezogenen Bereich zwischen dem ersten Bereich und dem zweiten Bereich zu definieren, und
Befestigen des ersten Bereichs und des zweiten Bereichs aneinander, um Seitennähte (54) zu bilden, wobei das Schneiden mindestens eines Teils (104) der Bahn das Schlitzen der Bahn (100) umfasst.

6. Verfahren gemäß Anspruch 5, wobei kein Teil der Bahn (100) mit dem Schnittteil entfernt wird.

7. Verfahren gemäß Anspruch 5, welches des Weiteren das Schneiden eines Ausschnitts (103) an einem inneren Ende des Schnittbereichs (104) umfasst.

8. Verfahren gemäß einem der Ansprüche 5 bis 7, welches das Falten der Bahn (100) gegen eine Unterstützungsstruktur (130) umfasst.

9. Verfahren gemäß Anspruch 8, welches das Aufbringen mindestens eines Streifens (106) auf die gefaltete Bahn in dem mindestens einen ausgewählten Bereich (26) umfasst.

10. Verfahren gemäß Anspruch 8 oder 9, welches das Schneiden mindestens eines Teils der Bahn (104) umfasst, während die Bahn gefaltet ist.

11. Verfahren gemäß einem der Ansprüche 5 bis 10, welches des Weiteren das Befestigen einer absorptionsfähigen Struktur (60) an der Bahn (100) umfasst.

12. Verfahren gemäß Anspruch 11, welches das Befestigen der absorptionsfähigen Struktur (60) an der Bahn umfasst, während die Bahn (100) gefaltet ist.

13. Verfahren gemäß Anspruch 11, welches das Befestigen der absorptionsfähigen Struktur (60) an der Bahn (100) vor dem Falten der Bahn (100) umfasst.

14. Verfahren gemäß Anspruch 11, welches das Befestigen der absorptionsfähigen Struktur (60) an der Bahn (100) nach dem Falten der Bahn (100) umfasst.

15. Verfahren gemäß einem der Ansprüche 5 bis 14, wobei die Bahn (100) eine einzelne Hosenanordnung umfasst.

16. Verfahren gemäß einem der Ansprüche 5 bis 14, wobei die Bahn (100) eine kontinuierliche Bahn ist, die eine Vielzahl von Hosenanordnungen umfasst, die miteinander verbunden sind.

17. Verfahren gemäß Anspruch 16, welches des Weiteren das Falten der Bahn (100) gegen mindestens zwei Unterstützungsstrukturen (130, 140) umfasst, von welchen jede parallel zu einer Richtung (102) ist, in welcher die Bahn (100) befördert wird, wobei jede Maschinenrichtungsanordnung von Hosenanordnungen gegen eine einzelne Unterstützungsstruktur (130, 140) gefaltet wird.

18. Verfahren gemäß einem der Ansprüche 5 bis 17, welches das Befördern der Bahn (100) in einem Maschinenrichtungsanordnungsprozess umfasst.

19. Verfahren gemäß einem der Ansprüche 5 bis 17, welches das Befördern der Bahn (100) in einem Quermaschinenrichtungsanordnungsprozess umfasst.

## Revendications

1. Culotte (10) fabriquée à partir d'une bande (100), la culotte comprenant :
une enveloppe de vêtement (64), l'enveloppe de vêtement (64) comportant une région avant (22), une région arrière (24), une région d'entrejambe (26), un bord de ceinture avant (38), un bord de ceinture arrière (39), des jonctions latérales (54) reliant la région avant (22) à la région arrière (24), deux ouvertures pour les jambes (52) et des jambes pendantes (23), au moins une partie de chacune parmi la région avant (22), la région arrière (24), la région d'entrejambe (26) et les jambes pendantes (23) comprenant des parties de la bande (100), les ouvertures pour les jambes (52) étant formées par des coupures (104) le long de deux bords opposés transversalement (107) de la bande (100), et les jambes pendantes (23) ne comportant pas d'élastification autour d'une périphérie complète des ouvertures pour les jambes (52),
**caractérisée en ce que** :
les coupures (104) le long des bords opposés transversalement (107) de la bande (100) consistent en des fentes.

2. Culotte (10) selon la revendication 1, comprenant en outre un ruban (106) dans la région d'entrejambe (26), dans laquelle le ruban (106) occupe au plus un tiers de la longueur de l'enveloppe de vêtement (64) lorsque l'enveloppe de vêtement (64) est dans un état posé à plat, complètement étendu.

3. Culotte (10) selon la revendication 1 ou 2, comprenant en outre un ruban (106) dans la région d'entrejambe (26), dans laquelle le ruban (106) comprend un matériau à rétraction différée.

4. Culotte (10) selon l'une quelconque des revendications précédentes, comprenant en outre une structure absorbante (60) fixée à l'enveloppe de vêtement (64) dans la région avant (22), la région arrière (24) et/ou la région d'entrejambe (26).

5. Procédé de fabrication de la culotte (10) selon l'une quelconque des revendications précédentes, comprenant les étapes consistant à :
fournir une bande (100) possédant une première région et une deuxième région ;
découper au moins une partie (104) de la bande pour définir deux ouvertures pour les jambes (52), l'au moins une partie découpée (104) définissant en outre une séparation entre la première région et la deuxième région ;
contracter la bande (100) dans une zone sélectionnée (26) pour définir au moins une zone contractée entre la première région et la deuxième région ; et
fixer ensemble la première région et la deuxième région pour former des jonctions latérales (54), ladite étape consistant à découper au moins une partie (104) de la bande comprenant l'étape consistant à pratiquer des fentes dans ladite bande (100).

6. Procédé selon la revendication 5, dans lequel aucune partie de la bande (100) à l'intérieur de la partie découpée n'est retirée.

7. Procédé selon la revendication 5, comprenant en outre le découpage d'une découpure (103) à une extrémité intérieure de la partie découpée (104).

8. Procédé selon l'une quelconque des revendications 5 à 7, comprenant le pliage de la bande (100) contre une structure de support (130).

9. Procédé selon la revendication 8, comprenant l'application d'au moins un ruban (106) sur la bande pliée dans l'au moins une zone sélectionnée (26).

10. Procédé selon la revendication 8 ou 9, comprenant le découpage de l'au moins une partie (104) de la bande pendant que la bande est pliée.

11. Procédé selon l'une quelconque des revendications 5 à 10, comprenant en outre la fixation d'une structure absorbante (60) à la bande (100).

12. Procédé selon la revendication 11, comprenant la fixation de la structure absorbante (60) à la bande pendant que la bande (100) est pliée.

13. Procédé selon la revendication 11, comprenant la fixation de la structure absorbante (60) à la bande (100) avant le pliage de la bande (100).

14. Procédé selon la revendication 11, comprenant la fixation de la structure absorbante (60) à la bande (100) après le dépliage de la bande (100).

15. Procédé selon l'une quelconque des revendications 5 à 14, dans lequel la bande (100) comprend un seul groupe de culottes.

16. Procédé selon l'une quelconque des revendications 5 à 14, dans lequel la bande (100) est une bande continue comprenant une pluralité de groupes de culottes reliés les uns aux autres.

17. Procédé selon la revendication 16, comprenant en outre le pliage de la bande (100) contre au moins deux structures de support (130, 140), chacune étant parallèle à une direction (102) dans laquelle la bande (100) est acheminée, dans lequel chaque ensemble de groupes de culottes dans le sens machine est plié contre une seule structure de support (130, 140).

18. Procédé selon l'une quelconque des revendications 5 à 17, comprenant l'acheminement de la bande (100) dans un processus d'assemblage dans le sens machine.

19. Procédé selon l'une quelconque des revendications 5 à 17, comprenant l'acheminement de la bande (100) dans un processus d'assemblage dans le sens travers.
